# EUROPEAN PATENT SPECIFICATION

(11) **EP 1 836 111 B1**
(45) Date of publication and mention of the grant of the patent: **11.01.2012**
(21) Application number: 05803577.5
(22) Date of filing: 15.11.2005
(51) Int. Cl.: B65G 1/137, G06Q 10/00

(54) **APPARATUS FOR STORING AND FOR INDEPENDENT DISPENSING OF A PLURALITY OF PACKAGES**
VORRICHTUNG ZUM AUFBEWAHREN UND UNABHÄNGIGEN ABGEBEN MEHRERER PAKETE
APPAREIL POUR LE RANGEMENT ET LA DISTRIBUTION DE CONDITIONNEMENTS

(30) Priority: 15.11.2004 GB 0425142; 16.03.2005 GB 0505395
(43) Date of publication of application: 26.09.2007
(60) Divisional application: 11164853.1
(73) Proprietor: Arx Limited, St. Albans, Herts AL1 5LU (GB)
(72) Inventor: CONNIER, Jean-Louis, 72200 La Flèche (FR); NOGUES, Guillaume, 72200 Mareil-sur-Loir (FR); KATRITZKY, Rupert, 98000 Monaco (MC); HASENFRATZ, Luc, Hemel Hempstead Hertfordshire HP3 8EW (GB)
(74) Representative: Samuels, Adrian James
(86) International application number: PCT/GB2005/004384
(87) International publication number: WO 2006/051329

(56) References cited:
- EP-A- 1 275 597
- EP-A- 1 347 423
- DE-A1- 19 857 282
- FR-A- 2 837 479
- CHRIST ET AL: "Neue Ideen zum automatischen Kommissionieren" F + H. FORDERN UND HEBEN, VEREINIGTE FACHVERLAG, MAINZ, DE, vol. 35, no. 12, 1 December 1985 (1985-12-01), pages 910-911, XP002096847 ISSN: 0341-2636

## Description

This invention relates to apparatus for the automated storage and retrieval of small packages, particularly although not exclusively pharmaceutical packages.

Automated pharmacies, whereby pharmaceuticals and the like are stored, retrieved and dispensed without the need for human intervention after the initial request, are well known in the art. Typically such systems comprise a robot arm in communication with a database of the locations of the packages on a rack of shelving. When a request for a particular package is received, the robot arm moves to the correct location, retrieves the desired package and transfers it to a dispensing chute.

The maximum output rate of such systems is limited by the speed with which the robot arm can move and the sweep area of the arm - i.e. the area which the arm is required to cover. Clearly, the output rate may be increased by providing a second or further robot arms but this adds significantly to the cost and also adds to the complexity of the system as they must be co-coordinated to avoid obstructing one another.

Shelf space tends to be allocated dynamically in such arrangements as packages are put into the system, so that there is no correlation between the type of drug in the package and its location on the shelving. Consequently, there is no correlation between the type of drug and the time taken for it to be retrieved and dispensed.

There is a recognition in the art that some types and sizes of pharmaceutical packages are required much more often than others. These are known in the art as "fast movers". In one proposal disclosed in WO 03/010073, separate areas of shelving are provided for fast movers each with its own dedicated robot arm on the other side of the shelf for retrieving packages solely from that area to allow fast movers to be retrieved more quickly without interfering with the main robot arm.

However, the Applicant has appreciated that there are some significant limitations associated with this arrangement. Firstly, retrieval is still carried out by a robot arm which must be translated in two-dimensions to the appropriate point on the shelf to pick the pack before it can be dispensed and thus, although quicker than the conventional arrangement, dispensing times are still of the same order.

Secondly, the additional robot arms add significantly to the expense as mentioned above. Furthermore this extra expense is incurred for each module which is added as a particular installation is expanded.

FR2837479 discloses a storage and dispensing apparatus including channels with an actuating mechanism to eject, stored packages and a conveyor to transport the ejected packages.

It is an object of the present invention to improve upon such arrangements. The present invention provides an apparatus for storing and dispensing a plurality of packages as claimed in claim 1. The invention also provides a method for storing and dispensing a plurality of packages as claimed in claim 22.

Typically, each channel comprises an independent ejection mechanism.

Thus it will be seen by those skilled in the art that in accordance with the invention a storage and dispensing system is provided in which the second storage region does not rely on a further robot arm but rather each channel of the second region can output independently to the conveyor. This allows extremely rapid, parallel output of packages. Furthermore, it is easily scalable since the second, channel region can easily be produced in modular form, e.g. with a common conveyor or with each module having a conveyor for moving packages to the edge of the module and then either to the conveyor of an adjacent module or to a downstream dispensing system such as a further conveyor or dispensing chute.

The Applicant has also appreciated that the arrangement of a conveyor to receive packs ejected from the channel region means that the conveying means may act as a kind of temporary buffer to receive a plurality of different packs which are required together, e.g. as a single prescription or to meet an order for a ward round. By collecting the packages together before then conveying them together to the delivery system or location, a significant time saving is made over prior art arrangements in which each pack is conveyed individually in turn to the delivery point. This is of course of particular significance where there is a relatively long separation between the storage and retrieval apparatus and the ultimate destination - e.g. a pharmacy front counter. This will normally be desirable so as to free up working space for pharmacy personnel and to isolate the noise of the apparatus as it operates.

If the apparatus of the invention is arranged at a sufficiently elevated height, even after packages have been ejected and have fallen from the channels of the channel region to end up on the conveyor, they will still be at a sufficient height for the final delivery mechanism e.g. a chute. In preferred embodiments, however, lifting means are provided for lifting packages ejected from the channel region and onto the conveyor to an increased height. For example, in preferred embodiments the lifting means is provided adjacent to the channel region and is arranged so that packages ejected from the channels are conveyed to the lifting means by the conveying means. This again is advantageous over the arrangement with one or more robot arms for retrieval since it enhances the flexibility of how the apparatus may be configured and where it may be placed for delivery to a final delivery point, but without significantly increasing the cost.

Furthermore, a single lift may be provided to serve a plurality of channel modules; indeed further channel modules may be added to an existing system without necessarily the need to add a further lift. It has also been appreciated that the lifting means may act as a buffer too, in addition to or instead of the conveying means, to allow several packages to be collected and delivered together which represents an enhancement in output speed as compared to delivering the packages to the final delivery point one at a time.

The lift is preferably arranged to eject the packages thereon to a final delivery system such as a chute. Indeed, it is envisaged that in some embodiments where it would be desirable to have a number of different final delivery points - e.g. to serve a plurality of operators- the lift could transport the packages to one of a plurality of final delivery mechanisms. For example, dispensing chutes could be arranged at different heights along the travel of the lifting means so that the packages on the lifting means may be selectively dispensed to a desired final delivery point by raising them to the appropriate level to eject them.

The lifting means should be arranged so as to raise packages to a different height and either deliver them to a further delivery system such as an output chute or present them for retrieval. Many designs are possible. One possibility is to arrange for the conveying means, or at least part thereof, to be able to be raised and lowered as required. This has some advantages including space efficiency. Alternatively, a simple raised platform could be provided.

Ejection of packs at the desired height can also be effected in a number of ways. The raising platform could be inclined away from the desired exit side of the lift with a door or gate attached to the platform itself or on a wall of the apparatus facing the platform. Alternatively the platform could be tilted only when the destination height is reached, with or without a door/gate etc. In one possible embodiment the platform is suspended from two opposite sides so that tilting can be induced by differences in the height of suspension between the two sides.

In accordance with the invention there is at least some overlap in the paths taken by packages dispensed from the first and second regions respectively. Indeed, a common conveying means may be used from which packages from both regions are conveyed to a final delivery point. This might be helpful in realising the benefits of the buffer function of the conveyor since the packages required to make up a single order may be distributed between the first and second regions. However, in preferred arrangements, the packs dispensed from the first and second regions are separate from one another for at least part of their paths since this helps to avoid problems with bottle necks occurring and thereby slowing the overall output rate.

Apparatus in accordance with the invention could be arranged so that packages ejected from the channel region fall directly onto the conveying means. This is, however, not preferred since it gives rise to the possibility of damage being caused to the package or its contents as a result of the force of the fall. In preferred embodiments, therefore, a soft region for receiving falling packages is provided from which the packages can fall or slide a further short distance slide onto the conveyor. Many ways of implementing this are possible although it is presently preferred to provide a loosely-slung sheet of material - e.g. fabric that can cushion the falling package without too great a tendency for packages to bounce off.

In some preferred embodiments two distinct channel regions may be provided, which could have a common soft landing area or preferably individual ones, but feeding a common conveying means.

The provision of two or more discrete channel regions is a general feature of a set of preferred embodiments. Of course, in accordance with the invention one of these channel regions will be the second, channel region of the apparatus of the invention referred to above. It will therefore be arranged so as to be filled by the storage and retrieval device serving the first region of shelving. Any additional channel regions could be served for filling by the same device. Alternatively, one or more additional storage and retrieval devices could be provided; or one or more of the further channel regions may be arranged to be filled manually. This would allow a manual backup in the event of malfunctioning of the other channel region and/or allow the inputting of packages into the system to be supplemented manually in order to speed up input.

Where a plurality of channel regions is provided, these are preferably served by a common lifting means although for reasons of capacity or backup, more than one lift may be provided each of which is able to serve each of the channel regions.

The ejection means associated with the individual channels may take any convenient particular form, for example, the foremost region of each channel could be arranged to tip or release the package in that region out of the front of the channel whilst preventing the other packages from being released; and thereafter allowing the remaining packages to slide down the channel so that a new package is received in the foremost region.

In a particularly preferred set of embodiments the ejection means comprises a pivoting member arranged to pivot from a first position in which it receives a package to a second position in which it ejects the package, wherein said member is arranged so as to be unable to receive a further package in said second position.

This is beneficial as it allows, simply and reliably, the ejection of just one package at a time without the need for complicated arrangements involving synchronised actuators or actuators operating for precisely defined times.

The remaining packs will be arranged so that another is received by the pivoting member when one has been ejected and the member is returned to its first position. This could be under any convenient force such as a spring but preferably the channel is inclined so that gravity causes the packs to move along the channel; towards the pivoting member.

The pivoting member could be arranged to prevent packages being received thereby additionally in the first position. This would require a third position in which the pivoting member was charged with the package which would then be 'cradled' from both sides. Preferably however the pivoting member is arranged to be able to receive a package in the first position.

The pivoting member preferably comprises a front and rear arms arranged such that in the first position the front arm prevents ejection of the pack and in the second position the front arm is retracted sufficiently for the pack to be ejected. The second arm could then, preferably, be retracted in the first position. It will be appreciated that the resulting arrangement is somewhat analogous to an escapement mechanism.

The ejection means could be operated by any suitable operating arrangement such as a motor, electromagnet, solenoid etc. However the Applicant has devised a particularly convenient and simple arrangement. In accordance with preferred embodiments the ejection means is operated by thermally responsive actuating means, means being provided to heat the thermally responsive actuating means to cause it to adopt a different configuration thereby moving the ejection means. The thermally responsive actuating means could comprise a construction where the requisite movement is derived from simple expansion, e.g. a bimetallic element. Preferably though it comprises a shape memory material. Such materials are well known *per se* and share the characteristic that they adopt a previously formed shape when heated to a predetermined temperature. Although some plastics exhibit this phenomenon it is preferred in the present case to use a shape memory metal. Conveniently the heating means comprises means for passing an electric current through at least a part of the shape memory metal element itself so that the element self-heats.

The ejection means, e.g. the preferred pivoting member, could be arranged to return to a standby position under gravity or could be actively moved to such a position but preferably it is resiliently biased towards its standby position. In the preferred embodiment comprising a pivoting member, the standby position translates to the first position.

In another alternative method of ejection, pressure may be applied to a stack of packages in a channel from the rear end, that is to say the end from which they are filled, in order to overcome a retaining force of some sort and allow the foremost package to be ejected. One advantage envisaged with this arrangement is that in the event of malfunction, the storage and retrieval device might be adapted to be able to apply the necessary pressure to a given channel to eject a package therefrom. This would allow the apparatus to continue to operate, albeit at a reduced output rate, if such a malfunction were to occur.

Although not essential, it is generally preferred for the channels in the channel region of the invention to slope towards the front to assist ejection of packages therefrom. The Applicants have appreciated that this creates an unused space at the bottom rear of the channel region and in another preferred feature, therefore the invention comprises providing shelving of the type employed in the first region of the apparatus in the lower rear part of the second, channel region. The advantage this brings in enhancing compactness could be significant in small installations. It should be appreciated that this is a further distinction over arrangements of the sort disclosed in WO 03/010073 in which the channel regions are provided as separate modules which are merely placed adjacent to more traditional shelving.

An alternative use of this dead space would be to provide the conveying means and thus in at least some preferred embodiments, the conveying means of the invention is provided directly beneath the channel region. In another alternative, the dead space may be used to provide a dispensing chute to deliver packages to a final delivery point.

A yet further and preferred use of the dead space is to house an output chute for the first region of shelving. Especially conveniently the output chute may be arranged to deliver packages onto the conveying means of the channel region. This would allow the advantages discussed above relating to the conveying and/or lift means as a buffer to be extended to the whole of the shelving. The output chute could be instead of a main output chute for the first shelving region, but preferably it is in addition. Indeed more generally it is preferred to provide a plurality of possible outputs for the first region of shelving. This is advantageous as it minimises the average distance the storage and retrieval device must move and therefore increases its speed of operation.

It will be appreciated from the foregoing that in accordance with the invention a storage and retrieval device is used to fill the channels of a channel region for allowing the same packages to be output rapidly.

The robot arms of conventional automated pharmacies are controlled by a computer which maintains a database of the locations of each package on the shelving. The channel region of the invention could simply be incorporated into a system like this so that the central database keeps a record of those packages which are stored from and subsequently dispensed from the channel region. However, this does potentially bring with it some complications. For example, in ordinary shelving there is generally only one pack at each location, whereas in the channel system there will be normally be a plurality of packages in each channel; this could cause difficulties in controlling software. Similarly the channel will normally be a FIFO (first in; first out) system which the controlling software may not easily be able to cater for. Thirdly, by the nature of fast movers stored in the channel region, it is especially disadvantageous to require frequent interrogation of the main database.

According to some preferred embodiments therefore the apparatus comprises communication means adapted to allow communication between the storage and retrieval device and the individuals channels or groups thereof. Suitable means include radio, microwave, ultrasonic or visual light or, preferably, infra-red transmission. The actual information communicated may be adapted to suit the particular implementation but could include identifying information about a package being put into a channel, the number and/or type of packages held in a given channel, error codes etc. Thus such communication might allow stock level control at least for fast movers to be managed locally - i.e. remotely from the central database. For example where a package of a given type is stored in both the first and second regions it could allow a decision as to the region in which to store a newly entered package.

The channel could pass information to the storage and retrieval device as to its stock level e.g. to direct the latter to place a package in a different channel if it is full. Indeed each channel could have a memory to allow it to keep its own mini-inventory and to request replenishment by the storage and retrieval when its items have been dispensed from the channel. In some preferred embodiments each channel has its own power supply such as a battery.

Another advantageous feature which the Applicant has devised and which may be used as appropriate comprises providing indicating means on each of the channels in the channel region so as to be visible to a user operating the apparatus manually in the event of a breakdown. In a convenient example of this feature a simple light could be lit for each of the channels from which a package must be picked to fulfill a given order. Alternatively a display such as a liquid crystal display (LCD) could be provided. This means that even if the ejection system of the channel region were to stop functioning, orders can still be fulfilled quickly by hand as a user is told from which channels to pick and does not need to be able to see the actual contents of the channels. Of course more complicated information could be conveyed to a user by such visual means, for example operational errors, stock required, mismatches between actual and planned numbers of packs, low stock levels etc. In one non-limiting example a pair of LEDs, e.g. red and green are provided through which several messages may be conveyed depending on the illumination or rate of flashing of the two LEDs.

The channels in the channel region will normally be arranged so as to dispense packages automatically at the front upon receipt of an appropriate command. Preferably each is further provided with means for allowing manual ejection e.g. in the event of a malfunction. This could be associated with the normal dispensing mechanism and also allow removal of packages at the front of the channel but the Applicant has realised that it is beneficial to provide means for allowing manual removal of the packages in a channel from the rear of the channel. This means that user access to the space in front of the channel region, which is usually kept small for optimising the use of space, is not required. There will usually be more space at the rear of the channel region since space is needed there to allow the storage and retrieval device to operate.

The channels in the channel region may have fixed dimensions. These need not all be the same and the distribution of channel dimensions can be chosen to fit the normal distribution of package sizes. However in some preferred embodiments at least some of the channels have at least one variable dimension. In preferred examples, the width of the channels is variable. This allows huge flexibility for the contents of a given channel to be changed depending upon prevailing requirements. The adjustment to the channel dimension could be one carried out manually e.g. with display means on each channel indicating that an adjustment is required and possibly also the nature of the adjustment; but preferably means are provided for altering the channel automatically. For example a small servo motor could be provided to effect the adjustment. This has the advantage of allowing frequent adjustments so that the contents of a given channel can be assigned dynamically.

Control of channel adjustments could be directed from a central computer but equally could be controlled by communication between the storage and delivery device and the channel itself as is described above. For example the storage and retrieval device could pass information as to the size of package it is holding to the channel to allow the channel to adjust its size accordingly.

One advantageous use of the apparatus of the preferred embodiments of the invention is to use some or all of the channels as a temporary hold for particular orders such as whole prescriptions which are required together. Prescription orders are often given with a reasonable degree of advance notice either because they are regular repeats or because a customer intends to return to collect them. This means that the whole prescription can be made up, stored in one or more of the channels and dispensed substantially simultaneously. Such a use particularly benefits from the preferred automatic adjustment of channel dimensions since the types of packages which are stored in channels used for this purpose will change rapidly.

In conventional automated pharmacies it is normal for the single robot arm to be controlled by a single microcomputer such as a personal computer (PC). As installations get larger and are operated by more personnel this creates a bottleneck.

Certain preferred embodiments of the invention will now be described, by way of example only, with reference to the accompanying drawings in which:
Fig. 1 is a schematic view of a first shelving region of an embodiment of the invention;
Fig. 2 is a schematic diagram showing a second channel region for use with the embodiment of Fig. 1;
Fig. 3 is a perspective view of an adjustable channel for use with the invention;
Fig. 4 is a view of the channel of Fig. 3 rotated 180º;

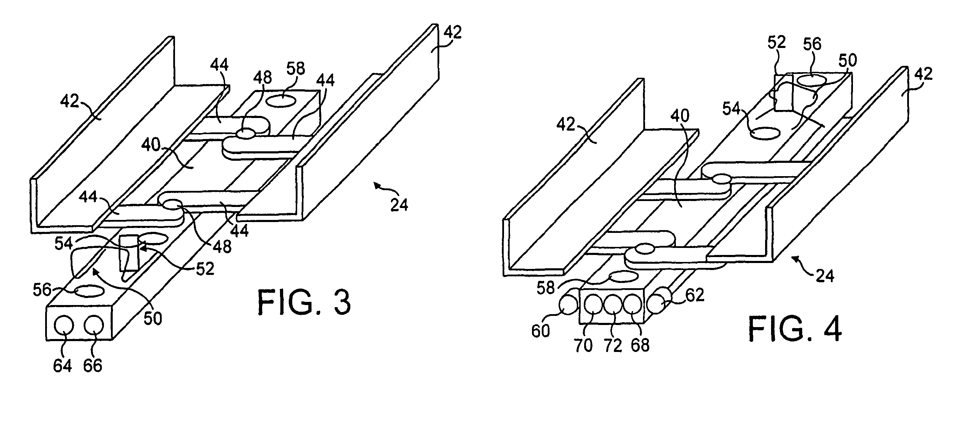

Fig. 4 is a view of the channel of Fig. 3 rotated 180º;

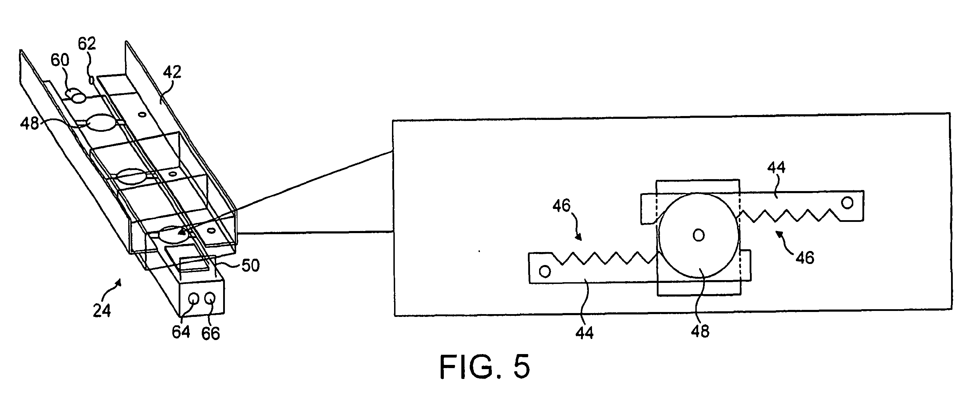

Fig. 5 is a view of the channel of Figs. 3 and 4 with enlarged detail showing the operating mechanism;

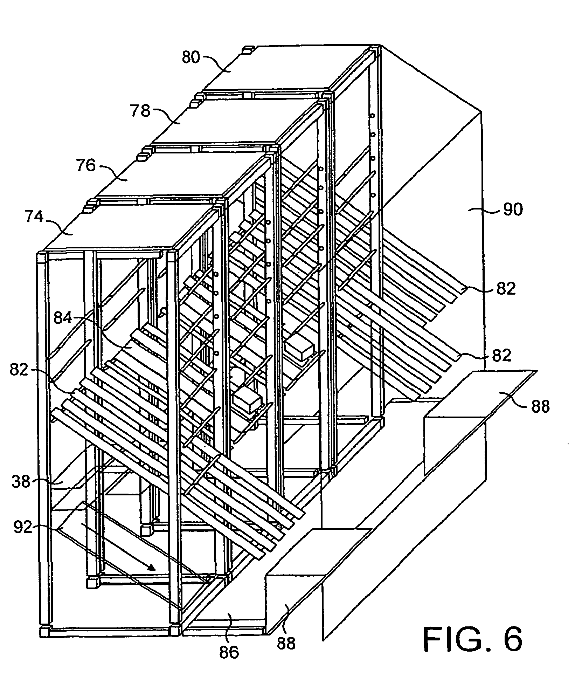

Fig. 6 is a schematic view of the channel region of a second embodiment of the invention;

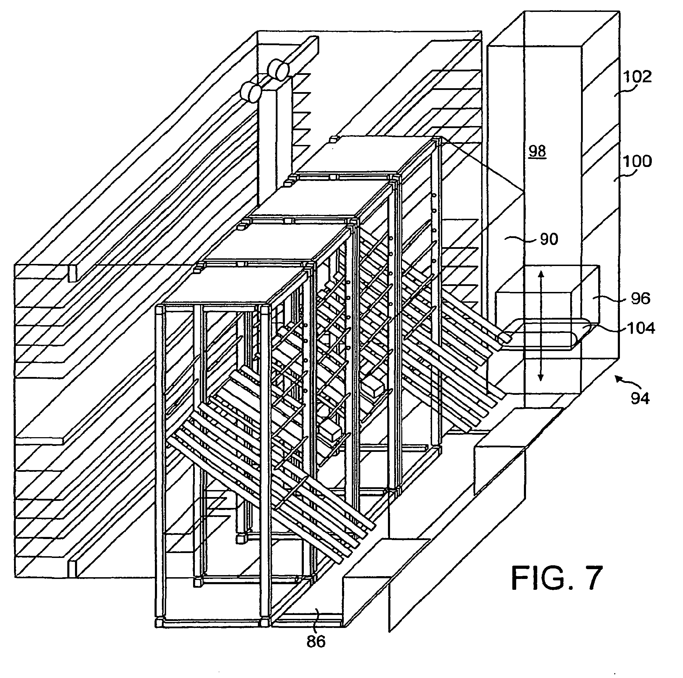

Fig. 7 shows the channel region of Fig. 6 in situ;

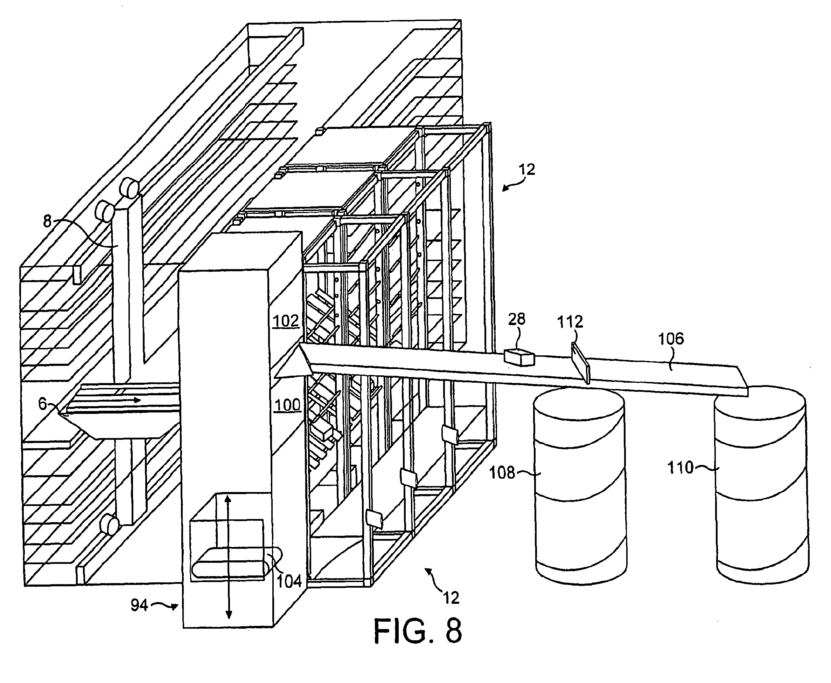

Fig. 8 is a schematic view of another embodiment of the invention;

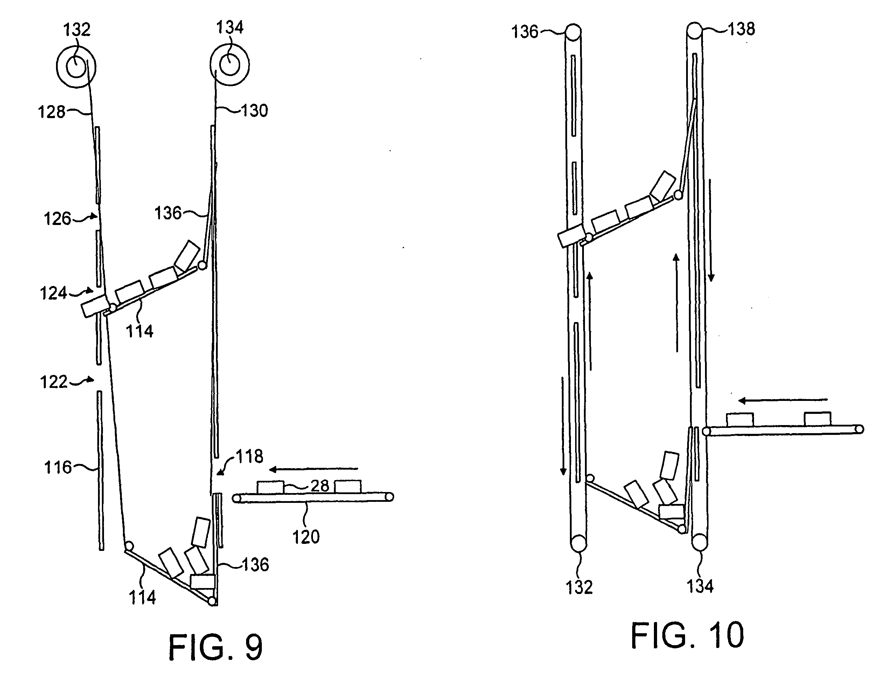

Fig. 9 is a sectional view through one embodiment of a lifting arrangement;
Fig. 10 is a sectional view through another embodiment of a lifting device;

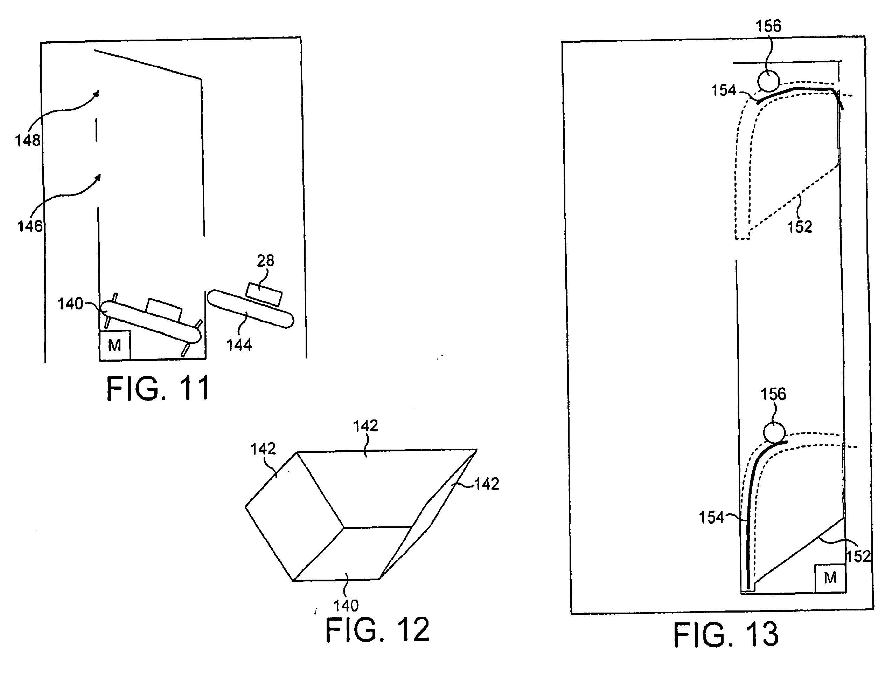

Fig. 11 is a schematic view of another embodiment of a lifting device;
Fig. 12 is a view of part of the lifting device of Fig. 11;
Fig. 13 is a sectional through yet another embodiment of a lifting device;

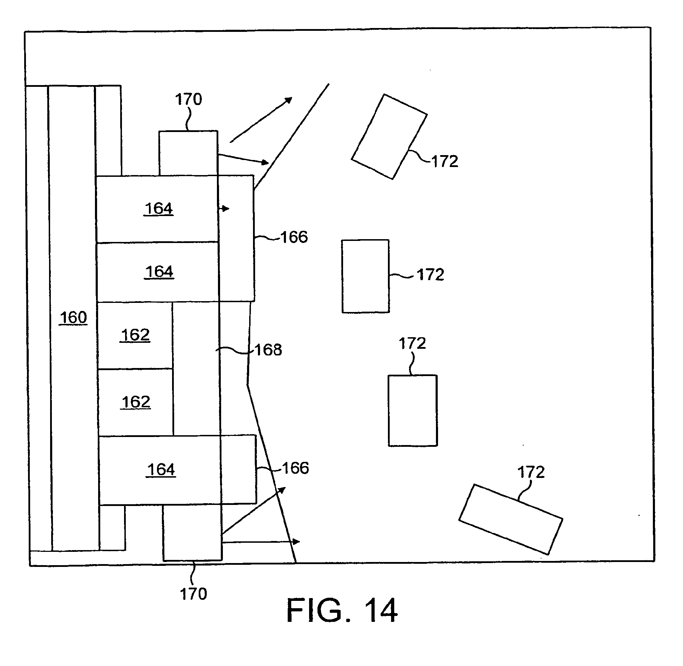

Figs. 14-19 are schematic views of various pharmacy layouts which may be used in accordance with the invention;

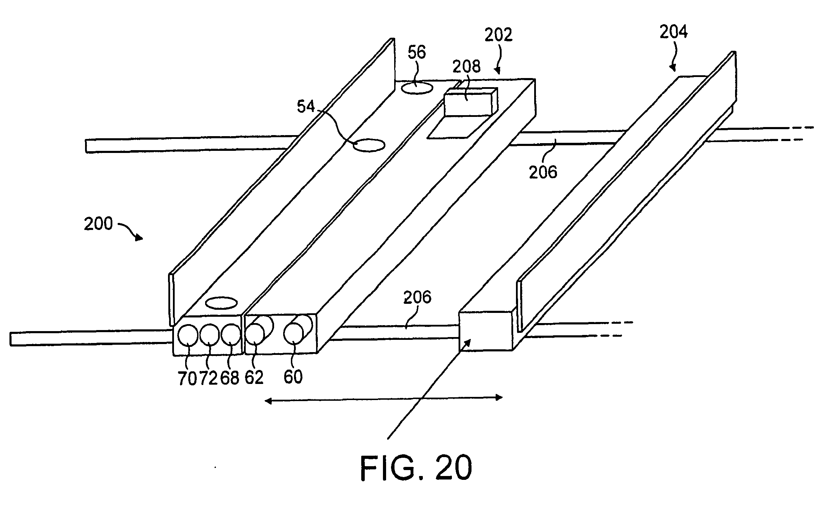

Fig. 20 is a schematic view of a further embodiment of a channel;

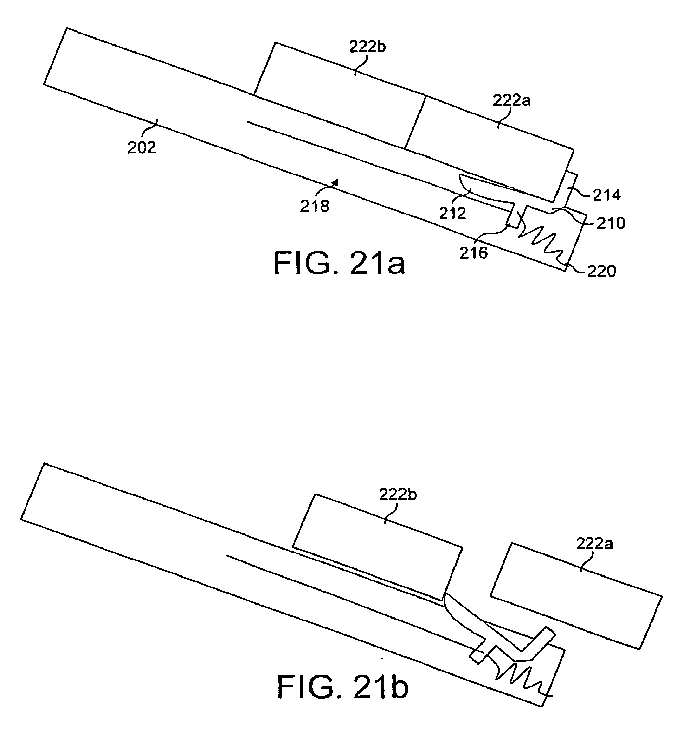

Fig. 21a is a schematic sectional view of the an ejector mechanism of the channel of Fig. 20 in a standby mode; and
Fig. 21b is a view similar to Fig. 21a showing an ejection mode.

The embodiments of the invention described herein below are all automated pharmacy systems of the type generally known in the art for storing large numbers of pharmaceutical packages and dispensing them on demand in response to an order. The dimensions and other physical characteristics of the various elements to be described are therefore to be inferred from this use.

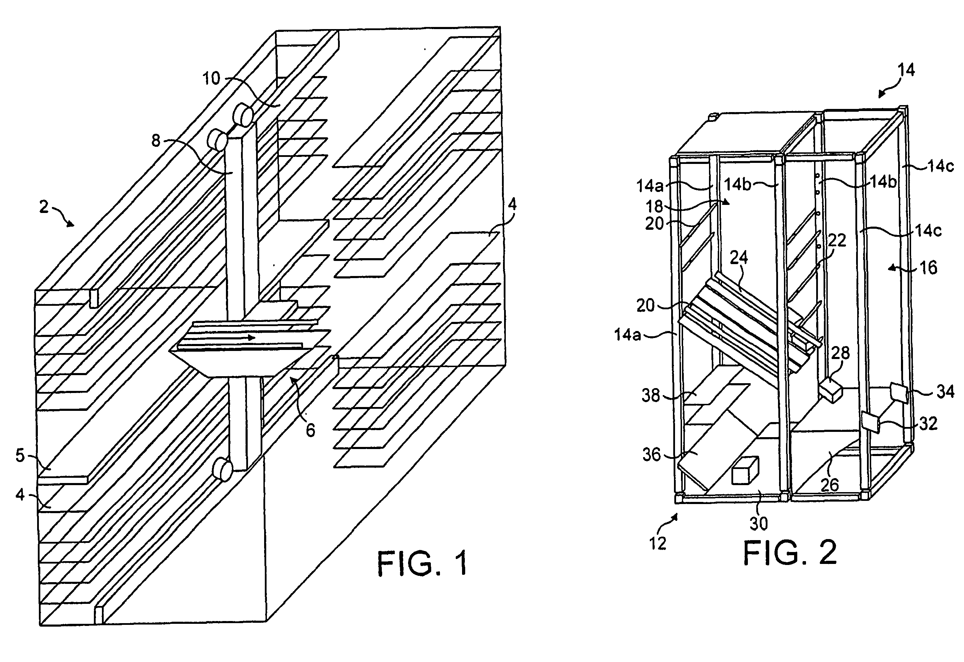

Fig. 1 shows a first, generally conventional region of shelving designated generally by the reference numeral 2 and comprising a vertically spaced array of elongate shelves 4 facing inwardly on either side of a corridor. A conveyor belt 5 is provided onto which packages are placed when they are received into the pharmacy and from which they may be picked up and placed onto the shelves 4. This runs the length of the shelving region 2 so that a large number of packages can be input into the system at once and thereafter placed onto the shelves 4 when the system is not occupied fulfilling orders for dispensing. Although shown transparent for reasons of clarity in this Figure, the shelves 4 and side walls of the corridor onto which the shelves back are normally closed at least sufficiently to prevent the packages passing through.

A robot arm assembly 6 is provided between the two sets of shelves 4 on either side of the aisle. The arm 6 is mounted to a vertical beam 8 on a sliding carriage that can be translated up and down the vertical beam 8 and horizontally towards and away from the shelves 4. The vertical beam 8 is itself mounted for translational sliding movement on a pair of horizontally extending guide rails 10. The arrangement therefore allows the robot arm 6 to move freely in the x, y or z direction. Furthermore the mounting of the arm assembly 6 onto the vertical beam 8 is such as to permit a 180 degree rotational movement so that shelves 4 on both sides can be accessed. The arm assembly 6 can therefore access any part of the shelving region 2. Although not shown in the Figures, as is well known in the art the arm assembly 6 comprises a pair of jaws which may be opened and closed and raised and lowered to allow packages to be manipulated. The robot arm 6 is depicted only schematically in this Figure although in practice it may be any of the type generally known in the art which are able to retrieve packages of a variety of sizes, shapes and weights from these shelves 4 move them to a different position and thereafter release them.

It will be observed that in Figure 1 part of the foremost side wall is left free of conventional shelves 4. Instead, in accordance with the present invention this area is provided with a second, channel region for frequently required packages otherwise known as "fast movers". Some elements of the channel region are shown schematically in Fig. 2.

Turning therefore to Fig. 2, it will be seen that the channel region, designated by the numeral 12 generally comprises an outer frame 14 which defines a front section 16 and a rear section 18 which is somewhat deeper than the front section 16 but of the same width.

The frame 14 comprises three pairs of parallel vertical struts, 14a, 14b and 14c at the rear, middle and front respectively. A series of horizontal cross members 20 extend between the rearmost vertical struts 14a. Similar horizontal cross members 22 extend between the centre vertical struts 14b.

A series of adjacent channel structures 24 extend between one of the rear cross members 20 and one of the essential cross members 22. The central cross member 22 supporting the channel structures 24 is lower than the corresponding rear cross member 20 so that the channel structures 24 slope downwardly from rear to front e.g. at an angle of about 30°. Only one layer of channels 24 is shown in Fig. 2 for clarity but in practice there will be a number of parallel layers at different heights. In practice therefore a large proportion of the volume of the rear section 18 of the frame will be filled with the array of parallel channels 24.

The lower, front end of the channels 24 open into the front part 16 of the channel region 12. The front part 16 is generally open but towards the bottom there is a soft landing platform 26 which could for example consist of a piece of fabric stretched across the frame, in order to cushion the fall of packages 28 which fall from the channels 24. The soft platform 26 is angled downwardly in a rearward direction so that packages 28 gently fall off it onto a conveyor belt which is provided at the bottom of the rear part 18 of the module, directly beneath the layers of channels 24. The conveyor 30 is operable in either lateral direction to enable a package 28 to be conveyed either to the left or right edge of the module 12.

The front vertical struts 14c are provided with an infrared transmitter and receiver pair 32, 34 just above the soft landing platform 26 which transmit and receive signals to and from the individual channel assemblies 24 as will be explained in greater detail below. To the rear of the conveyor 30 is an inclined chute surface 36, the purpose of which will be explained below, and a some further shelves 38. It will be appreciated therefore that rather than the lower rear space, i.e. that which is left by the slope of the channels 24, being wasted; the space is usefully used by accommodating the additional output chute 36 and additional shelving capacity 38.

Although shown separated, in practice the channel module 12 in Fig. 2 is positioned in the space in the shelving region 2 mentioned above. Further channel modules may be placed adjacent to it depending on requirements.

Figs. 3, 4 and 5 show one possible detailed configuration of one of the channel assemblies 24. Figs. 3 and 4 are views from the front and rear of the channel respectively and are foreshortened to allow all of the salient features to be easily seen. Each channel 24 comprises an elongate base member 40 which is fixed to the front and rear cross members 20, 22. The base member 40 supports a pair of L-shaped side wall members 42. Each side wall member 42 has two horizontally extending arms 44 which, as is seen in the enlarged portion of Fig. 5, have toothed edges 46 which engage cogs 48 to allow the arms 44 to be translated horizontally in opposite directions in a double rack and pinion drive arrangement as shown in Fig. 5. This allows the two side walls 42 to be easily moved together and apart to alter the width of the channel which allows it to accommodate differently sized packages. The walls could be adjusted manually by an operator (i.e. a service engineer would not be required) or they could be moved by the robot arm 6. In another possible embodiment (not shown) the cogs 48 could be replaced by servo motors to allow the channels to be adjusted automatically.

Towards the front of the channel 24 is a raised wire pack stop 50 which prevents the packages sliding off the front end of the channel. Immediately behind the pack stop 50 is an ejector flap 52 which is operable to flip up and push a package lying on it over the pack stop thereby causing the package to slide off the front end of the channel.

Immediately behind the ejector 52 is a pack detector 54 which is able to sense when there is a package at the front of the channel behind the pack stop. A similar detector 56 is provided at the very front of the channel in order to allow passing of a dispensed package to be detected. A third pack detector 58 is at the rear of the channel to allow the detection of newly entered packages at the back of the channel.

Also at the back of the channel is a manual ejection button 60 which operates the ejector 52 when pressed by a user; and a mechanical ejection lever 62 which enables the ejector 52 to be operated by hand in the event of a power failure. The manual knobs 60, 62 therefore allow a user to eject a package from the normal, front end of the channel in the event of various types of malfunction. As will be appreciated by returning to Fig. 1, being able to operate the manual ejection mechanisms from the rear of the channel is beneficial since it avoids the need to provide user access to the front of the apparatus. There is a reasonable amount of space behind the channel module 12 when installed as this is required for the robot arm 6 to operate.

Turning back to Fig. 3 on the front face of the channel assembly 24 there is a light emitting diode (LED) 64 which is used to indicate to a user in the event of a malfunction of the apparatus that a package should be picked from that particular channel in order to fulfill a given order. Next to the display LED 64 is an infrared emitter and receiver 66 which is the means by which the channel assembly 24 communicates with the central computer in normal use for receiving ejection commands and for returning status information.

An infrared emitter and receiver 68 is provided on the rear end of the channel assembly 24 to allow direct communication with the channel 24 - e.g. during maintenance or in the event of a malfunction. It could also be used in other embodiments for routine communication with the robot arm 6. The rear end face of the channel also has a display LED 70 which operates in the same way as the front LED 64 to allow packages to be picked or selected from the rear. However this LED 70 comprises independent red and green elements which through various combinations of illumination and flashing at different rates can convey a number of operational messages to a user for example not only that a pack is to be picked, but perhaps that a pack must be input, that multiple packs must be input, that stock is close to or at empty, that there is an input or output error, or that the channel has more or less stock than it is supposed to have. It is of course apparent that the particular messages and form of display may be chosen by a user to suit a given implementation.

Lastly the rear face provides a channel identifier device 72 which is arranged to transmit a signal identifying the channel in response to an interrogating signal from the robot arm 6. This allows the robot arm to ensure that it is by the correct channel.

Operation of the embodiment set out above will now be described. In operation, pharmaceutical packages are entered into the system at a suitable entry point (not shown). The robot arm 6 then picks each package up one at a time, and sends suitable identifying information to a controlling computer which returns a command to the robot arm 6 telling it where to place the package. In alternative arrangements the scanning step may be carried out by a user (so-called semi-automatic mode). In accordance with the embodiment of the invention described here, the package could be placed on one of the conventional shelves 4. Alternatively however, if the package is a so called "fast mover" it may be placed into one of the channels 24 of the channel module 12. The robot arm 6 will first interrogate the channel 24 to elicit an identifying signal and so verify it has the correct channel. It will then place the package at the top, rear of the channel 24 so allowing the package to slide down the channel to the pack stop 50 if the channel is empty or the rear of the stack of packages already in there. The arm 6 may adjust the width of a channel 24 if necessary by moving one of the sidewalls 42 thereof. It might also check with the channel via the central computer, or in alternative embodiments by means of the infra-red transmitter/receiver arrangement 68, that there is space for an additional package (e.g. if the rearmost pack detector 58 is uncovered).

When a request for a particular pack is received, this will be processed by the central computer which will either command the robot arm to retrieve it from one of the shelves 4 if it is not a fast mover, or if it is a fast mover the computer will send a dispense instruction to the appropriate channel 24 by means of an infra-red signal from the infra-red transmitter 32 (Fig. 2) at the front of the channel module to the receiver 66 at the front of the channel. This causes the ejector 52 operate by flipping up and tipping the package over the pack stop 50. The package 38 will then fall off the end of the channel and onto the soft landing area 26; and from there onto the conveyor 30. For packages that are frequently required in multiples, several channels may be used to store them so that orders requiring multiples of a such a pack may be fulfilled very quickly by dispensing from multiple channels simultaneously.

If further packages are required to make a prescription these may be dispensed simultaneously either from the channel module 12 or by means of the arm 6 which can pick a package from the appropriate shelf 4,38 and place it onto the output chute 36 of the channel module. Once all the packages of the prescription have been dispensed onto the conveyor 30, this may be operated to carry them to a delivery chute or the like so that a customer can receive them all together much more quickly that if each pack is conveyed to the delivery point separately.

In the event of a partial malfunction of the channel module 12 - e.g. a loss of main power, drugs may still be dispensed by hand or the robot arm 6 by using either the manual electric or mechanical ejectors 60,62. Furthermore this is significantly facilitated by LEDs being lit on the channels from which drug packs are required. The LEDs could be powered from a reserve battery since their current consumption is very low. This means that an operator does not need to be able to see the packs themselves in the channel 24.

A further embodiment of the invention is shown in Fig. 6. In this embodiment, there are four separate channel modules 74-80 which all differ to some extent from the channel module 12 described with reference to Fig. 2. The two outer modules 74, 80 have significantly deeper channel assemblies 82 than those provided in the inner modules 76, 78 which are of similar size to those in the first embodiment. The longer channels 82 of the outer modules are particularly useful for very fast movers thanks to their high capacity.

In contrast to the first embodiment, the individual modules 74-80 do not have their own conveyor system but rather they all use a common conveyor belt 86 located in front of or below the modules. The longer, outer channels 82 feed packages on to the conveyor 86 by means of respective soft landing platforms 88 which are opposite the ends of the channel. Similar soft landing platforms are provided for the shorter, inner channels 84 directly beneath the end of these channels, but these are omitted from the Figure for the sake of clarity. The whole of the channel region comprising the four modules 74-80 is protected by a screen 90 which prevents packs falling out of the apparatus.

As in the first embodiment, an output chute 92 for the robot arm is provided beneath one of the modules 74 although of course in this embodiment it is longer since the conveyor 86 is forward of the module. A chute 92 is shown only in one of the modules in the Figure, but in practice all of them are provided with similar chutes This is advantageous in reducing the average distance the robot arm is required to travel between retrieving a package from the ordinary shelving and depositing it in an output chute. Also in common with the first embodiment, some additional shelving 38 is provided in the dead space below the sloping channels 82, 84.

Fig. 7 shows the channel region which features in Fig. 6 integrated into the rest of the ordinary shelving 4 which is as depicted in Fig. 1. Operation of this embodiment up to the packages 28 being carried on the conveyor 86 is exactly the same as in the first embodiment and will therefore not be described again here. Again the conveyor 86 can conveniently act as a buffer to collect all of the packages which are required together in a single order. This allows them to be output the user altogether which significantly improves the output speed as compared to outputting each of them individually when it is considered that conventionally a package would need to be conveyed all the way to the final delivery point before transport of the subsequent package is commenced.

Newly shown in Fig. 7 is a lift system designated generally by the numeral 94. It broadly comprises a lift platform 96 which is driven vertically within a lift shaft 98 from the base of the shaft to one of two raised openings 100, 102. A small conveyor belt 104 forms the base of the lifting platform 96. The platform may or may not have side walls and may if necessary have a door or gate both on the side facing the channel region conveyor 86 and the openings 100, 102 respectively. As will be clearly seen, in operation packages on the static conveying means 86 are conveyed onto the conveyor 104 of the lifting platform 96 when the latter is at its lowest position and the lift platform is then raised until the base conveyor 104 is level with one of the openings 100, 102. The packages on the base conveyor 104 may then simply be discharged onto a delivery chute or slide coupled to the respective opening 100, 102 by operating the conveyor to move the packages forward and off the platform 96.

A further embodiment of the invention is shown in Fig. 8. In fact, this embodiment is simply a combination of three of the channel modules 12 shown in Fig. 2 with the ordinary shelving region shown in Fig. 1. A lift module 94 identical to that described with reference to Fig. 7 is shown at one end of the channel region. Also shown specifically in this embodiment is a delivery conveyor 106 coupled with the upper of the two lift openings 102. The delivery conveyor 106 serves two spiral final delivery chutes 108, 110 and a swinging diverter bar 112 is provided across the path of the conveyor 106 to allow a choice to be made as to which of the delivery chutes 108, 110 a particular package 38 will fall into.

The multiple outputs 100,102 of the lift module however allow simplification of the downstream conveyor system since it is not required to offer a selection of conveying paths. This also makes the controlling software less complex and permits a higher overall output rate.

Figs. 9-13 show various alternative configurations for the lifting module. Turning firstly to Fig. 9, there may be seen a schematic sectional view of one possible alternative lifting arrangement. In this arrangement, a lifting platform 114 is suspended for vertical movement within a shaft 116. The shaft 116 has a lower entry aperture 118 in the region of a conveyor 120 and three raised openings 122, 124, 126 for outputting packages. Of course having three openings is purely exemplary and any number may be provided as convenient. The lift platform 114 is suspended on two independently driven ropes or belts 128, 130 which are driven by respective motors 132, 134. One of the ropes 128 is hitched directly to the platform 114, whilst the second of the ropes 130 is hitched to a wall member 136 which is itself hingedly attached to the platform 114.

In use, packages 38 arrive on the conveyor 120 and are carried through the entrance aperture 118 and from there they fall into the wedge-shaped bucket formed by the lift platform 114 and the side wall 136. When all of the packages 28 for a given order have been collected, the two motors 132, 134 are operated to raise the platform 114. When the platform reaches the desired exit aperture e.g. the second of the three apertures 124 as shown in Fig. 9, the left hand motor 132 is stopped but the right hand motor 134 is made to continue running to shorten the corresponding rope 130 further so that the platform 114 is tilted up until the packages 38 slide off the platform and through the exit aperture 124 and onto a conveyor or chute. It will be appreciated from this that the lifting means may therefore act as a buffer.

Fig. 10 shows a lift arrangement which is very similar to Fig. 9 except that the motors 132, 134 are disposed at the bottom of the shaft with pulleys 136, 138 at the top of the shaft. This arrangement may be beneficial in allowing easier access to the motors 132, 134 for maintenance. It also helps to maintain a close registry between the platform and the output openings.

A further lift arrangement is shown in Fig. 11 and Fig. 12. In this arrangement a lift "bucket" is formed with an inclined conveyor belt 140 as its base and three sloping side walls 142 to contain the packages. In the lower configuration shown in Fig. 11, packages 28 are received from a conveyor from the shelving regions into the bucket. The bucket may then be raised to one of two exit apertures 146, 148 and the conveyor 140 run to eject to the packages into a chute or onto a further conveyor.

A further lift arrangement is shown in Fig. 13. In this arrangement, a lift box 150 has a sloping floor 152 and is closed at the front by a roller shutter 154 operated by motor 156. The shutter 154, when down as shown in the lower part of Fig. 13, leaves an opening at the top of the box 150 to allow packages to be put into it. The lift box 150 may then be raised to a higher level and the shutter motor 156 operated to raise the shutter and thereby allow packages contained in the lift box 150 to slide off the sloping floor 152.

Various possible layouts for the various modules will now be given with reference to Figs. 14-19. One possible layout is seen in Fig. 14. In this, a first conventional shelving region 160 with associated robot arm (not shown) extends across the width of the pharmacy. On one side of the conventional shelving region is a channel region comprising five channel modules 162, 164. Two of the modules are normal channel modules 162 and the other two are deep modules 164. Examples of deep and shallow modules may be seen in the embodiment described with reference to Figs. 6 and 7.

In front of each of the deep channel modules 164 is a pack dropping area 166 which acts to soften the landing of the packages and to direct them backwardly onto a conveyor 168 which is common to all five modules and runs beneath the deep modules 164 and in front of the shallow modules 162. At either end of the conveyor 168 is a lift module 170, each of which has two exits. This arrangement means that any one of four operator stations 172 may be served by the apparatus by directing a particular prescription to the appropriate lift module 170 and then to the appropriate exit of that lift.

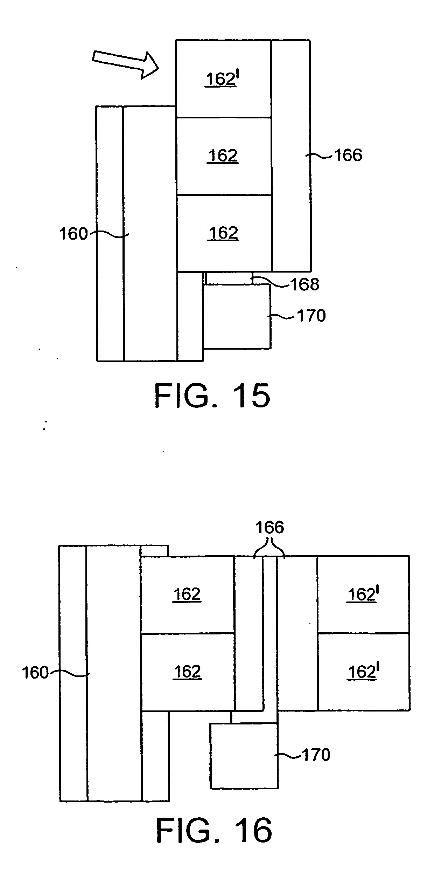

Another possible layout is shown in Fig. 15. In this arrangement a conveyor 168 runs underneath three channel modules 162, 162' and a pack dropping area 166 is provided in front of the modules. The modules 162, 162' are therefore similar to those described with reference to Figure 2. However, it will be noted in this arrangement that only two of the channel modules 162 are within the area of the conventional shelving 160 and therefore accessible by the robot arm to fill them. The third module 162' is directly accessible from the rear to allow it to be filled manually. This increases the overall input speed to the system which is possible.

Fig. 16 shows another possible layout. In this arrangement, there are two manually filled channel modules 162' opposite two modules 162 which are filled by the robot arm in the conventional shelving region 160. The opposite pairs of modules each have respective pack dropping areas 166 which pass the packages onto a common conveyor 168 which in turn passes them into the lift module 170. To save space in this layout it could be modified to have just a single dropping area for all the module 162 and 162', with the conveyor being provided under the leftmost modules 162 as in the previous embodiment.

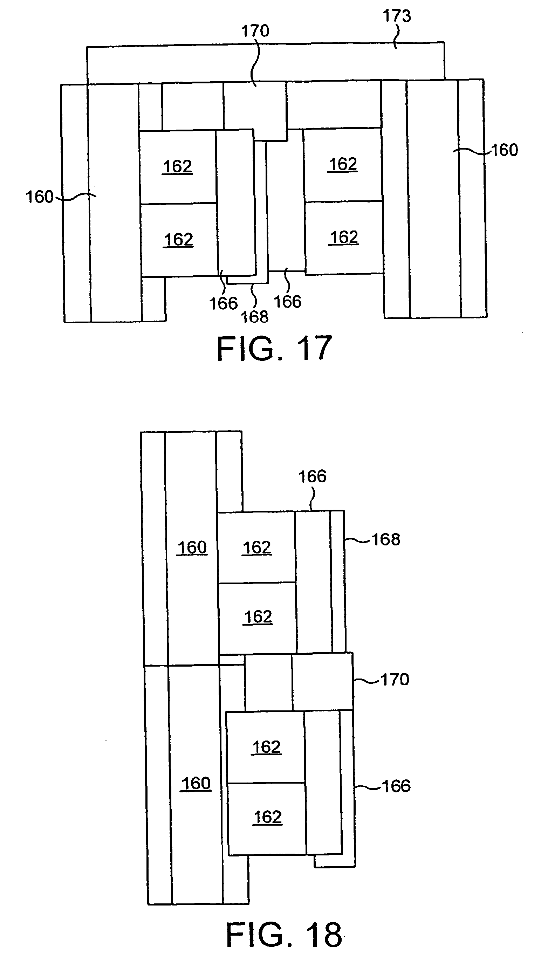

Fig. 17 shows a tandem layout which is similar to that in Fig. 16 but wherein a second shelving region 160 and associated robot arm fill the rightmost channel modules 162. This embodiment also has a further conveyor 173 between the two conventional shelving regions 160 which allows stock to be transferred between them and also allows packages retrieved from the shelving regions 160 (as opposed to the channel modules 162) to be placed into the lift 170. This is advantageous for example in allowing the lift 170 to act as a buffer to collect all of the packages required together to fulfill a prescription wherever in the system they may be stored.

Fig. 18 simply shows an arrangement in which two separate shelving regions 160 with associated robot arms feed two channel modules 162 each but, as in the previous layout, there is a common lift 170 which may act as a buffer for prescriptions. Again this embodiment could be modified to have the conveyor as part the modules 162 and so provided at the bottom thereof to preserve the modularity of preferred embodiments of the invention.

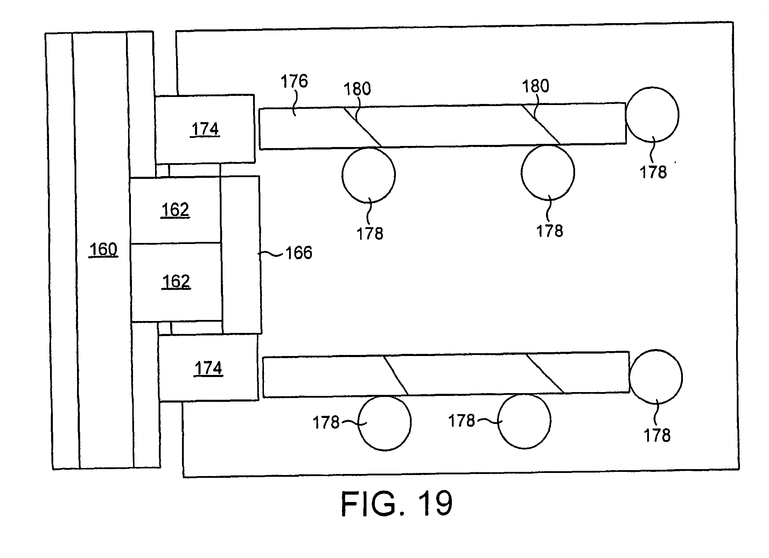

Finally, Fig. 19 shows an arrangement in which the storage part of the apparatus is arranged on the floor above the actual pharmacy counter from where it is required to issue the pharmaceutical packs. In this embodiment, there is a shelving region 160 with associated robot arm feeding two channel modules 162 as in previous embodiments. However, in contrast to previous embodiments, the conveyor 168 feeds into one of two holes 174 at either end of it which pass through the floor and open onto respective conveyors 176 which are towards ceiling level of the floor below and each of which feeds into three spiral chutes 178 down to a level at which the packages may be collected. As no lift module(s) is/are required, this represents a cost saving.

Swinging diverter bars 180 are provided above the conveyors 176 to allow the correct destination chute to be selected. This embodiment therefore allows six separate operators to receive packages at their own locations.

Fig. 20 shows a further embodiment of a channel 200 in accordance with the invention. In this embodiment the channel 200 comprises a main portion 202 broadly as described previously with reference to Figs. 3 to 5; and an auxiliary portion 204. The main and auxiliary portion 202, 204 are mounted on a pair of rails 206 so as to be manually slidable therealong towards and away from each other to allow adjustment of the channel width. This could be indicated by the LED 70 through an appropriate combination of flashes (e.g. red for widen, green for narrow). The pack detectors 54 to 58; ejection buttons 60,62; infrared detector and receiver 68. display LED 70 and channel identifier device 72 are as previously described.

However this embodiment incorporates a different ejector mechanism 208 described in more detail with reference to Figs. 21a and 21b. The ejector mechanism 208 comprises a hood-shaped member 210 which is pivotally mounted with respect to the channel 202. The pivoting member 210 comprises a base plate 212 and an upstanding wall 214. A shaft 216 extends down from the base plate 212 and provides the pivot point at its lower end. Attached to the shaft 216 above the pivot is one end of a shape memory wire 218. The other end of the wire 218 is anchored to the channel body 202. Also attached to the shaft 216 above the pivot point is a restoring spring 220.

Fig. 21a shows the normal, standby position in which the baseplate is substantially flush with the surface of the channel body 202 and a pack 222a is received on it so as to rest against the endwall 214. when an electrical current is passed through the shape memory wire 218 its electrical resistance causes it to heat up which in turn causes it to return to its 'hot' shape which is extended as compared to its ambient state. This extension is transmitted to the shaft 216 of the pivoting member causing it to tilt forward. This allows the foremost pack 222a to slide off the end of the inclined channel and so be ejected. At the same time however since the rear end of the base plate 212 is raised up the pack behind 222b cannot slide onto the pivoting member 210 and so cannot be ejected.

When current is removed from the wire 218 it will contract again and, aided by the spring 220, the pivoting member 210 returns to its standby position. This allows the next pack 222b to slide onto and be received by the pivoting member 210 ready itself to be ejected. It will be appreciated therefore that simple, reliable, one-at-a-time ejection of packs is achieved.

It will be appreciated by those skilled in the art that the embodiments described above are merely examples and that there are many possible variants within the scope of the invention.

## Claims

1. An apparatus for storing and dispensing a plurality of packages (28) comprising-a first (2;160) and second (12;74,76,78,80;162,164)-regions from which packages can be dispensed respectively, and a storage and picking device (6), the first region (2;160) having a plurality of shelves (4;38) accessible by the storage and picking device (6) and the second region (12;74,76,78,80;162,164) having a plurality of channels (24;82,84;200), each for receiving a plurality of packages and each of which comprises independent ejection means (52;208), wherein the storage and picking device (6) is adapted to be able to fill the channels (24;82,84;200) in the second region (12;74;76,78,80;162,164); the apparatus further comprising conveying means (30;86;168) associated with the second region (12;74,76,78,80;162,164) for removing packages (28) ejected from the second region (12;74,76,78,80;162,164); wherein the apparatus is arranged such that in use there is at least some overlap within apparatus between a first path taken by packages (22) dispensed from the first region (2;160) and a second path taken by packages dispensed from the second region (12; 74, 75, 78, 80; 162, 164).

2. Apparatus as claimed in claim 1 comprising lifting means (94;170) for lifting packages (28) ejected from the channel region (12;74,76,78,80;162,164) and onto the conveying means (130;86;168) to an increased height.

3. Apparatus as claimed in claim 2 wherein said lifting means (94;170) is provided adjacent to the channel region (12;74,76,78,80;162,164) and is arranged so that packages (28) ejected from the channels (24;82,84;200) are conveyed to the lifting means (94;170) by the conveying means (30;86;168).

4. Apparatus as claimed in claim 2 or 3 wherein said lifting means (94;170) is arranged to transport the packages (28) to one of a plurality of final delivery mechanisms.

5. Apparatus as claimed in any preceding claim comprising a common conveying means (30;86;168) for conveying packages (28) from both said first (2;160) and second regions (12;74,76,78.80;162,164) to a final delivery point.

6. Apparatus as claimed in any preceding claim comprising one or more discrete further channel regions (162') which are arranged for filling by the storage and picking device (6) and/or arranged to be filled manually.

7. Apparatus as claimed in claim 6 comprising a common lifting means (170) serving all of said channel regions (162,164).

8. Apparatus as claimed in any preceding claim wherein the ejection means (52;208) associated with the individual channels (24;82,84;200) are arranged to tip or release a package (28) received in a foremost part of the channel from the front of the channel whilst preventing any other packages received in the channel from being released; and thereafter allowing the remaining packages to slide down the channel so that a new package is received in the foremost part.

9. Apparatus as claimed in any preceding claim wherein the ejection means (208) comprises a pivoting member (210) arranged to pivot from a first position in which it receives a package (28) to a second position in which it ejects the package, wherein said member (210) is arranged so as to be unable to receive a further package in said second position.

10. Apparatus as claimed in any of claims 1 to 7 wherein the ejection means (52) associated with the individual channels (24;82,84) comprise means for applying pressure to a stack of packages (28) in the channel from the rear end in order to overcome a retaining force and allow the foremost package to be ejected.

11. Apparatus as claimed in any preceding claim wherein the channels (24;82,34;200) in the channel region (12;74,76,78,80;162,164) slope towards the front to assist ejection of packages (28) therefrom, further comprising in the lower rear part of the channel region a dispensing chute (36;92) for delivering packages to a final delivery point.

12. Apparatus as claimed in any of claims 10 wherein the channels (24;82,84;200) in the channel region (12;74,76,78,80;162,164) slope towards the front to assist ejection of packages (28) therefrom, further comprising in the lower rear part of the channel region an output chute (36; 92) for the first regions .

13. Apparatus as claimed in claim 12 wherein said output chute (36;92) is arranged to deliver packages (28) onto the conveying means (30;86;168) of the channel region (12;74,76.78,80;162,164).

14. Apparatus as claimed in any preceding claim comprising a plurality of possible outputs for the first region (2; 160).

15. Apparatus as claimed in any preceding claim comprising communication means (68,72) adapted to allow communication between the storage and picking device (6) and the individuals channels (24;82,84;200) or groups thereof.

16. Apparatus as claimed in any preceding claim comprising indicating means (64,70) on each of the channels (24;82,84;200) in the channel region (12;74,76,78,80;162,164) so as to be visible to a user operating the apparatus manually.

17. Apparatus as claimed in claim 16 wherein each of said channels (24;82,84;200) comprises a light (64,70) which is arranged to be lit for each of the channels from which a package (28) must be picked to fulfill a given order.

18. Apparatus as claimed in any preceding claim wherein each of the channels (24;82,84;200) in the channel region (12;74,76,78,80;162,164) is arranged so as to dispense packages (28) automatically at the front upon receipt of an appropriate command.

19. Apparatus as claimed in claim 18 wherein each channel (24;82,84;200) is further provided with means for allowing manual ejection.

20. Apparatus as claimed in claim 18 or 19 wherein each channel (24;82,84;200) comprises means for allowing manual removal of the packages (28) from the rear thereof.

21. Apparatus as claimed in any preceding claim wherein at least some of the channels (24;82,84;200) in the channel region (12;74,76,78,80;162,164) have at least one variable dimension and further comprising means for altering the channel automatically.

22. A method of storing and dispensing a plurality of packages, comprising:
storing packages in a first region (2;160) having a plurality of shelves (4;38);
storing packages in a second region (12;74,76,78,80;162,164) having a plurality of channels (24;82,84;200);
filling the shelves and picking packages from the shelves (4;38)in the first region (2;160) with a storage and picking device (16);
filling the channels (24;82,84;200) in the second region (12;74,76,78,80;162,164) with the same storage and picking device (6);
dispensing packages from the first region (2;160) to a first path, removing packages from the channels (24;82,84;200) in the second region (12;74,76,78,80;162,164) using independent ejection means (52,208), and conveying the packages ejected from the channels (24;82,84;200) along a second path,
wherein there is at least some overlap within the apparatus between the first and second paths.

## Patentansprüche

1. Vorrichtung zum Lagern und zur Abgabe einer Anzahl von Packungen (28) mit einem ersten (2; 160) und einem zweiten (12; 74, 76, 78, 80; 162, 164) Bereich, aus welchen jeweils Packungen abgegeben werden können, und einer Lager- und Auswahl- bzw. Sortiervorrichtung (6), wobei der erste Bereich (2; 160) eine Anzahl von Fächern bzw. Regalen (4; 38) aufweist, die durch die Lager- und Auswahlvorrichtung (6) zugänglich sind, und der zweite Bereich (12; 74, 76, 78, 80; 162, 164) eine Anzahl von Kanälen (24; 82, 84; 200) aufweist, die jeweils zur Aufnahme einer Anzahl von Packungen eingerichtet sind, und von denen jeder unabhängige Auswurfmittel (52; 208) aufweist, wobei die Lager- und Auswahlvorrichtung (6) dafür eingerichtet ist, die Kanäle (24; 82, 84; 200) in dem zweiten Bereich (12; 74; 76, 78, 80; 162, 164) füllen zu können; wobei die Vorrichtung ferner Fördermittel (30; 86; 168) aufweist, die dem zweiten Bereich (12; 74, 76, 78, 80; 162, 164) zugeordnet sind, um Packungen (28), die aus dem zweiten Bereich (12; 74, 76, 78, 80; 162, 164) ausgeworfen wurden, zu entfernen; wobei die Vorrichtung so eingerichtet ist, dass bei der Verwendung wenigstens etwas Überlappung in der Vorrichtung zwischen einem ersten Weg, der durch Packungen (28), die aus dem ersten Bereich (2; 160) abgegeben wurden, genommen wird, und einem zweiten Weg, der durch Packungen, die aus dem zweiten Bereich (12; 74, 76, 78, 80; 162, 164) abgegeben wurden, genommen wird, besteht.

2. Vorrichtung nach Anspruch 1, mit Hubmitteln (94; 170) zum Heben von Packungen (28), die aus dem Kanalbereich (12; 74, 76, 78, 80; 162, 164) ausgeworfen wurden, und auf die Fördermittel (30; 86; 180) auf eine größere Höhe.

3. Vorrichtung nach Anspruch 2, bei der die Hubmittel (94; 170) neben dem Kanalbereich (12; 74, 76, 78, 80; 162, 164) bereitgestellt und so eingerichtet sind, dass Packungen, die aus den Kanälen (24; 82, 84; 200) ausgeworfen wurden, durch die Fördermittel (30; 86; 168) zu den Hubmitteln (94; 170) gefördert werden.

4. Vorrichtung nach Anspruch 2 oder 3, bei der die Hubmittel (94; 170) dafür eingerichtet sind, die Packungen (28) zu einem aus einer Anzahl von Endabgabemechanismen zu transportieren.

5. Vorrichtung nach einem der vorstehenden Ansprüche, mit gemeinsamen Fördermitteln (30; 86; 168) zum Fördern von Packungen (28) aus sowohl dem ersten (2; 160) als auch dem zweiten Bereich (12; 74, 76, 78, 80; 162, 164) zu einem Endabgabepunkt.

6. Vorrichtung nach einem der vorstehenden Ansprüche, mit einem oder mehreren getrennten weiteren Kanalbereichen (162') die dafür eingerichtet sind, durch die Lager- und Auswahlvorrichtung (6) gefüllt zu werden und/oder die dafür eingerichtet sind, manuell gefüllt zu werden.

7. Vorrichtung nach Anspruch 6, mit gemeinsamen Hubmitteln (170), die alle der Kanalbereiche (162, 164) bedienen.

8. Vorrichtung nach einem der vorstehenden Ansprüche, bei der die Auswurfmittel (52; 208), die den einzelnen Kanälen (24; 82, 84; 200) zugeordnet sind, dafür eingerichtet sind, eine Packung (28), die in einem vordersten Teil des Kanals aufgenommen wurde, aus der Vorderseite des Kanals abzukippen bzw. freizugeben, während verhindert wird, dass sämtliche anderen Packungen, die in dem Kanal aufgenommen wurden, freigegeben werden; und um es hiernach den verbleibenden Packungen zu ermöglichen, den Kanal hinabzurutschen, so dass eine neue Packung in dem vordersten Teil aufgenommen wird.

9. Vorrichtung nach einem der vorstehenden Ansprüche, bei der die Auswurfmittel (208) ein Schwenkelement (210) aufweisen, das dafür eingerichtet ist, aus einer ersten Position, in welcher es eine Packung (28) aufnimmt, in eine zweite Position, in welcher es die Packung auswirft, zu schwenken, wobei das Element (210) dafür eingerichtet ist, dass es ihm unmöglich ist, in der zweiten Position eine weitere Packung aufzunehmen.

10. Vorrichtung nach einem der Ansprüche 1 bis 7, bei der die Auswurfmittel (52), die den einzelnen Kanälen (24; 82, 84) zugeordnet sind, Mittel zum Anlegen eines Drucks auf einen Stapel von Packungen (28) in dem Kanal von dem rückwärtigen Ende aus aufweist, um eine Rückhaltekraft zu überwinden und es der vordersten Packung zu ermöglichen, ausgeworfen zu werden.

11. Vorrichtung nach einem der vorstehenden Ansprüche, bei der die Kanäle (24; 82, 84; 200) in dem Kanalbereich (12; 74, 76, 78, 80; 162, 164) sich zu der Vorderseite neigen, um einen Auswurf von Packungen (28) hieraus zu unterstützen, und die ferner in dem unteren rückwärtigen Teil des Kanalbereichs einen Auswurfschacht (36; 92) zur Abgabe von Packungen an einen Endabgabepunkt aufweist.

12. Vorrichtung nach einem der vorstehenden Ansprüche, bei der die Kanäle (24; 82, 84; 200) in dem Kanalbereich (12; 74, 76, 78, 80; 162, 164) sich zu der Vorderseite neigen, um einen Auswurf von Packungen (28) hieraus zu unterstützen, und die ferner in dem unteren rückwärtigen Teil des Kanalbereichs einen Auswurfschacht (36; 92) für den ersten Bereich aufweist.

13. Vorrichtung nach Anspruch 12, bei der der Ausgabeschacht (36; 92) dafür eingerichtet ist, Packungen (28) auf die Fördermittel (30; 86; 168) des Kanalbereichs (12; 74, 76, 78, 80; 162, 164) abzugeben.

14. Vorrichtung nach einem der vorstehenden Ansprüche, mit einer Anzahl möglicher Ausgaben für den ersten Bereich (2; 160).

15. Vorrichtung nach einem der vorstehenden Ansprüche, mit Kommunikationsmitteln (68, 72), die dafür eingerichtet sind, eine Kommunikation zwischen der Lager- und Auswahlvorrichtung (6) und den einzelnen Kanälen (24; 82, 84; 200) oder Gruppen hiervon zu ermöglichen.

16. Vorrichtung nach einem der vorstehenden Ansprüche, mit Anzeigemitteln (64; 70) auf jedem der Kanäle (24; 82, 84; 200) in dem Kanalbereich (12; 74, 76, 78, 80; 162, 164), um durch einen Benutzer, der die Vorrichtung manuell bedient, sichtbar zu sein.

17. Vorrichtung nach Anspruch 16, bei der jeder der Kanäle (24; 82, 84; 200) ein Licht (64, 70) aufweist, welches dafür eingerichtet ist, für jeden der Kanäle beleuchtet zu werden, aus denen eine Packung (28) entnommen werden muss, um eine vorgegebene Reihenfolge einzuhalten.

18. Vorrichtung nach einem der vorstehenden Ansprüche, bei der jeder der Kanäle (24; 82, 84; 200) in dem Kanalbereich (12; 74, 76, 78, 80; 162, 164) dafür eingerichtet ist, Packungen (28) automatisch an der Vorderseite auf den Empfang des geeigneten Befehls hin abzugeben.

19. Vorrichtung nach Anspruch 18, bei der jeder Kanal (24; 82, 84; 200) ferner mit Mitteln zum Ermöglichen eines manuellen Auswurfs versehen ist.

20. Vorrichtung nach Anspruch 18 oder 19, bei der jeder Kanal (24; 82, 84; 200) Mittel zum Ermöglichen einer manuellen Entfernung der Packungen (82) aus der rückwärtigen Seite hiervon aufweist.

21. Vorrichtung nach einem der vorstehenden Ansprüche, bei wenigstens einige der Kanäle (24; 82, 84; 200) in dem Kanalbereich (12; 74, 76, 78, 80; 162, 164) wenigstens eine variable Dimension aufweisen und ferner Mittel zum automatischen Verändern des Kanals aufweisen.

22. Verfahren zum Lagern und Abgeben einer Anzahl von Packungen, das umfasst:
Lagern von Packungen in einem ersten Bereich (2; 160) mit einer Anzahl von Fächern bzw. Regalen (4; 38);
Lagern von Packungen in einem zweiten Bereich (12; 74, 76, 78, 80; 162, 164) mit einer Anzahl von Kanälen (24; 82, 84; 200);
Füllen der Fächer und Auswählen von Packungen aus den Fächern (4; 38) in dem ersten Bereich (2; 160) mit einer Lager- und Auswahlvorrichtung (16);
Auffüllen der Kanäle (24; 82, 84; 200) in dem zweiten Bereich (12; 74, 76, 78, 80; 162, 164) mit der gleichen Lager- und Auswahlvorrichtung (6);
Abgeben von Packungen aus dem ersten Bereich (2; 160) an einen ersten Weg, Entfernen von Packungen aus den Kanälen (24; 82, 84; 200) in dem zweiten Bereich (12; 74, 76, 78, 80; 162, 164) unter Verwendung unabhängiger Auswurfmittel (52, 208) und Fördern der Packungen, die aus den Kanälen (24; 82, 84; 200) ausgeworfen wurden, entlang einem zweiten Weg,
wobei wenigstens etwas Überlappung in der Vorrichtung zwischen dem ersten und dem zweiten Weg besteht.

## Revendications

1. Appareil pour stocker et distribuer une pluralité d'emballages (28) comportant une première (2; 160) et une deuxième (12; 74; 76; 78; 80; 162; 164) régions à partir desquelles des emballages peuvent être distribués respectivement, et un dispositif de stockage et de prélèvement (6), la première région (2; 160) ayant une pluralité d'étagères (4; 38) accessibles par l'intermédiaire du dispositif de stockage et de prélèvement (6) et la deuxième région (12; 74; 76; 78; 80; 162; 164) ayant une pluralité de goulottes (24; 82; 84; 200), chacune étant destinée à recevoir une pluralité d'emballages et chacune d'elles comportant des moyens d'éjection (52; 208) indépendants, dans lequel le dispositif de stockage et de prélèvement (6) est adapté pour pouvoir remplir les goulottes (24; 82; 84; 200) de la deuxième région (12; 74; 76; 78; 80; 162; 164); l'appareil comportant en outre des moyens d'acheminement (30; 86; 168) associés à la deuxième région (12; 74; 76; 78; 80; 162; 164) pour retirer des emballages éjectés depuis la deuxième région (12; 74; 76; 78; 80; 162; 164) ; dans lequel l'appareil est conçu de telle sorte que, en fonctionnement, il y a au moins un recouvrement à l'intérieur de l'appareil entre un premier trajet emprunté par les emballages (28) distribués depuis la première région (2; 160) et un deuxième trajet emprunté par les emballages distribués depuis la deuxième région (12; 74; 76; 78; 80; 162; 164).

2. Appareil selon la revendication 1, comportant des moyens de levage (94; 170) pour lever des emballages éjectés de la région des goulottes (12; 74; 76; 78; 80; 162; 164) et sur les moyens d'acheminement (30; 86; 168) jusqu'à une hauteur accrue.

3. Appareil selon la revendication 2, dans lequel lesdits moyens de levage (94; 170) sont agencés à proximité adjacente de la région des goulottes (12; 74; 76; 78; 80; 162; 164) et sont conçus de sorte que des emballages (28) éjectés des goulottes sont acheminés vers les moyens de levage (94; 170) par les moyens d'acheminement (30; 86; 168).

4. Appareil selon l'une des revendications 2 ou 3, dans lequel lesdits moyens de levage (94; 170) sont conçus pour transporter les emballages jusqu'à un mécanisme parmi une pluralité de mécanismes de livraison finale.

5. Appareil selon l'une quelconque des revendications précédentes comportant un dispositif d'acheminement commun (30; 86; 168) pour acheminer des emballages (28) à la fois depuis ladite première et ladite deuxième régions jusqu'à un point de livraison finale.

6. Appareil selon l'une quelconque des revendications précédentes comportant une ou plusieurs régions de goulottes (162') supplémentaires discrètes qui sont conçues pour être remplies par le dispositif de stockage et de prélèvement (6) et/ou conçues pour être remplies manuellement.

7. Appareil selon la revendication 6 comportant des moyens de levage communs (170) desservant toutes lesdites région de goulottes (162; 164).

8. Appareil selon l'une quelconque des revendications précédentes dans lequel les moyens d'éjection (52; 108) associés aux goulottes individuelles (24; 82; 84; 200) sont conçus pour décharger ou libérer un emballages (28) reçu dans une partie située la plus en avant de la goulotte à partir de l'avant de la goulotte tout en empêchant tout autre emballages quelconque reçu dans la goulotte d'être libéré ; et permettant ensuite aux emballages restants de glisser vers le bas dans la goulotte de sorte qu'un nouveau emballages est reçu dans la partie située la plus en avant.

9. Appareil selon l'une quelconque des revendications précédentes dans lequel les moyens d'éjection (208) comportent un élément pivotant (210) conçu pour pivoter à partir d'une première position dans laquelle il reçoit un emballages (28) jusqu'à une deuxième position dans laquelle il éjecte le emballages, ledit élément (210) étant conçu de manière à être incapable de recevoir un emballages supplémentaire dans ladite deuxième position.

10. Appareil selon l'une quelconque des revendications 1 à 7 dans lequel les moyens d'éjection (52) associés aux goulottes individuelles comportent des moyens pour appliquer une pression à une pile de emballages (28) dans la goulotte à partir de l'extrémité arrière afin de surmonter une force de retenue et de permettre l'éjection du emballages situé le plus en avant.

11. Appareil selon l'une quelconque de revendications précédentes dans lequel les goulottes (24; 82; 84; 200) dans la région des goulottes penchent vers l'avant afin d'aider à l'éjection des emballages (28) depuis celles-ci ; comportant de plus, dans la partie arrière inférieure de la région des goulottes, une glissière de distribution (36; 92) pour délivrer des emballages à un point de livraison finale.

12. Appareil selon l'une quelconque de revendications 1-10 dans lequel les goulottes (24; 82; 84; 200) dans la région des goulottes penchent vers l'avant afin d'aider à l'éjection des emballages (28) depuis celles-ci ; comportant de plus, dans la partie arrière inférieure de la région des goulottes, une glissière de distribution (36; 92) pour la première région.

13. Appareil selon la revendication 12 dans lequel ladite glissière de sortie (36; 92) est conçue pour délivrer des emballages (28) sur les moyens d'acheminement communs (30; 86; 168) de la région des goulottes (12; 74; 76; 78; 80; 162; 164).

14. Appareil selon l'une quelconque des revendications précédentes comportant une pluralité de sorties possibles pour la première région (2 ; 160).

15. Appareil selon l'une quelconque des revendications précédentes comportant des moyens de communication (68 ; 72) adaptés pour permettre une communication entre le dispositif de stockage et de prélèvement (6) et les goulottes individuelles (24; 82; 84; 200) ou des groupes de celles-ci.

16. Appareil selon l'une quelconque des revendications précédentes comportant des moyens (64 ; 70) d'indication sur chacune des goulottes dans la région des goulottes (24; 82; 84; 200) de manière à être visibles pour un utilisateur faisant fonctionner le appareil manuellement.

17. Appareil selon la revendication 16 dans lequel chacune desdites goulottes (24; 82; 84; 200) comporte une lumière (64 ; 74), laquelle est conçue pour être allumée pour chacune des goulottes depuis laquelle un emballages doit être prélevé afin de remplir une commande donnée.

18. Appareil selon l'une quelconque des revendications précédentes dans lequel chacune des goulottes (24; 82; 84; 200) dans la région des goulottes est conçue de manière à distribuer des emballages automatiquement à l'avant lors de la réception d'une instruction appropriée.

19. Appareil selon la revendication 18 dans lequel chaque goulotte (24; 82; 84; 200) est en outre munie de moyens pour permettre une éjection manuelle.

20. Appareil selon la revendication 18 ou 19 dans lequel chaque goulotte (24; 82; 84; 200) comporte des moyens pour permettre un retrait manuel des emballages à partir de l'arrière de celle-ci.

21. Appareil selon l'une quelconque des revendications précédentes dans lequel au moins certaines des goulottes (24; 82; 84; 200) dans la région des goulottes (12; 74; 76; 78; 80; 162; 164) ont au moins une dimension variable,, et comportant de plus des moyens pour modifier la goulotte automatiquement.

22. Procédé de stockage et de délivrance d'une pluralité de emballages, consistant à :
- stocker des emballages dans une première région (2 ; 160) comportant une pluralité d'étagères '4; 38) ;
- stocker des emballages dans une seconde région (12; 74; 76; 78; 80; 162; 164) comportant une pluralité de goulottes (24; 82; 84; 200) ;
- remplir les étagères et prélever des emballages à partir des étagères (4; 38) dans la première région (2; 160) avec un dispositif (6) de stockage et de prélèvement ;
- remplir les goulottes (24; 82; 84; 200) dans la deuxième région (12; 74; 76; 78; 80; 162; 164) avec le même dispositif (6) de stockage et de prélèvement ;
- délivrer des emballages à partir de la première région (2; 160) à un premier trajet, ;
- enlever des emballages des goulottes (24; 82; 84; 200) dans la deuxième région (12; 74; 76; 78; 80; 162; 164) en utilisant des moyens d'éjection (52; 208) indépendants, et
- acheminer les emballages éjectés depuis les goulottes (24; 82; 84; 200) le long d'un deuxième trajet, dans lequel il y a au moins un recouvrement à l'intérieur du appareil entre les premier et deuxième trajets.
